# EUROPEAN PATENT SPECIFICATION

(11) **EP 3 452 005 B1**
(45) Date of publication and mention of the grant of the patent: **30.12.2020**
(21) Application number: 17726033.8
(22) Date of filing: 04.05.2017
(51) Int. Cl.: A61K 8/29, A61Q 17/04, A61K 8/02

(54) **UV PROTECTIVE COMPOSITIONS AND THEIR USE**
VOR UV-STRAHLUNG SCHÜTZENDE ZUSAMMENSETZUNGEN
COMPOSITIONS PROTÉGEANT CONTRE LES ULTRAVIOLETS

(30) Priority: 05.05.2016 GB 201607831
(43) Date of publication of application: 13.03.2019
(73) Proprietor: Landa Labs (2012) Ltd., 7612301 Rehovot (IL)
(72) Inventor: LANDA, Benzion, 7405135 Nes Ziona (IL); ABRAMOVICH, Sagi, 4339320 Ra'anana (IL); DOR, Snir, 4906437 Petach Tikva (IL)
(74) Representative: Harrison IP Limited
(86) International application number: PCT/IB2017/052591
(87) International publication number: WO 2017/191585

(56) References cited:
- EP-A1- 2 229 931
- JP-A- H11 166 173
- JUM-SUK JANG ET AL: "Energy Band Structure and Photocatalytic Property of Fe-doped Zn 2 TiO 4 Material", BULLETIN OF THE KOREAN CHEMICAL SOCIETY, vol. 30, no. 12, 20 December 2009 (2009-12-20), pages 3021-3024, XP055386851, KR ISSN: 0253-2964, DOI: 10.5012/bkcs.2009.30.12.3021

## Description

### FIELD

The present disclosure relates to the field of protection from ultraviolet radiation, and more particularly, to UV protective compositions comprising doped or undoped zinc titanate crystals, to methods for preparing the same and uses thereof.

### BACKGROUND

Ultraviolet (UV) radiation is ubiquitous, the sun being the most common source of UV radiation although not the only source. As UV radiation can cause damage to people, animals and objects, compositions that provide protection from UV radiation are useful.

In the biological context, UV-protective compositions, *i.e.* compositions that reduce or block the transmission of UV rays, are commonly employed to protect against sunburn. Sunburn is a form of radiation burn resulting from an overexposure to UV radiation, typically from the sun, but also from artificial sources, such as tanning lamps, welding arcs, and ultraviolet germicidal irradiation.

Normal symptoms of sunburn in humans and other animals include reddening of the skin, general fatigue and mild dizziness. An excess of UV radiation can be life-threatening in extreme cases. Excessive UV radiation is considered to be the leading cause of non-malignant skin tumors, as well as increasing the risk of certain types of skin cancer.

Sunscreen compositions comprising UV protective agents are commonly used to prevent sunburn and are believed to prevent squamous cell carcinomas and melanomas. Furthermore, they have been reported to delay the development of wrinkles and additional age-related skin conditions.

Specifically, sunscreen compositions are topical compositions that include UV-protecting agents that absorb and/or reflect at least some of the sun's UV radiation on areas of skin exposed to sunlight, and thus reduce the effect of UV radiation on the skin. Depending on their mode of action, they are typically classified as chemical or physical sunscreens.

Chemical sunscreen compositions comprise organic compounds that absorb UV radiation to reduce the amount of UV radiation that reaches the skin. Being transparent to visible light and thereby being invisible when applied to the skin, chemical sunscreen compositions are popular for use. However, some organic compounds used in chemical sunscreen compositions have been found to generate free radicals which can cause skin damage, irritation and accelerated aging of the skin. Furthermore, organic materials may be absorbed into the skin, resulting in long-term detrimental health effects. Chemical sunscreen compositions may require the addition of a photostabilizer.

Physical sunscreen compositions reflect and absorb UV radiation. Known physical sunscreen compositions comprise particles of inorganic materials, mainly titanium oxide and/or zinc oxide. In order to obtain absorption and/or reflection of ultraviolet radiation over the full UVA and UVB range, relatively large particles are used. Due to the large particle size, such sunscreen compositions are viscous and opaque and tend to leave a white cast on the skin.

Many sunscreen compositions protect against UV radiation in the 280-315 nm range (UVB radiation) that causes sunburn, but do not against UV radiation in the 315-400 nm range (UVA radiation), which does not primarily cause sunburn but can increase the rate of melanoma and photodermatitis.

It is generally preferred that sunscreen compositions, when applied to the skin, are transparent to the eye. In order for physical sunscreen compositions to be transparent, the particles of inorganic material should be in the form of nanoparticles, which absorb and/or scatter UV light but not visible light, rendering them substantially transparent to the eye when applied to the skin. However, use of nanoparticles reduces the range of wavelengths absorbed by the inorganic materials. Some known sunscreen compositions therefore block both UVA and UVB radiation by use of a combination of different UV-absorbing or scattering materials, generally termed UV-protecting agents, each of which blocks radiation over a limited range of the UV spectrum.

Similarly, UV-protective compositions can benefit inert materials or objects that may be negatively affected by UV radiation. For instance, UV radiation can reduce the life-span of materials (*e.g.,* natural and synthetic polymers), and may modify colors of objects, especially in articles that are subjected to prolonged sun exposure, such as buildings or vehicles.

Various coatings are known to provide protection against UV radiation damage by blocking or reducing transmission of UV rays. Use of such coatings may in turn reduce the detrimental effect of UV radiation on a living animal. For example, use of said coating on optical lenses, thereby reducing the transmission of UV radiation, may reduce the incidence of UV-induced optical disorders such as cataract. Materials serving for the fabrication of windows incorporating or coated with suitable UV-protecting agents may reduce the transmission of UV radiation to subjects, plants, surfaces or objects shielded by such windows.

Japanese publication number H11166173 discloses an ultraviolet absorber formed from a composite metal oxide of the formula AB₂O₄ where A and B are each a metal element and O is oxygen. More specifically, A and B are different, and are selected from magnesium, manganese, iron, cobalt, copper, zinc, aluminium, chromium and titanium.

The present Applicant has disclosed sunscreen compositions comprising inorganic nanoparticles, *inter alia* in PCT Publication Nos. WO 2016/151537 and WO 2017/013633.

It would be desirable to have an effective UV protective composition, in particular providing broad-spectrum protection, and safe for use on living subjects.

### SUMMARY

The present disclosure, in at least some embodiments thereof, provides ultraviolet radiation protective compositions, such as, sunscreen compositions, that when applied to a surface provides protection from UV radiation, which in some embodiments have a broad spectrum UV protective activity, such compositions comprising zinc titanate (Zn₂TiO₄) crystals, optionally doped by iron atoms, as an ultraviolet-absorbing agent.

According to an aspect of some embodiments, there is provided a UV-protective composition comprising one or more zinc titanate crystals each independently having the chemical formula Zn₂Ti₍₁₋ₓ₎FeₓO₄, wherein x is between 0 and 0.1, as an ultraviolet-absorbing agent.

As employed herein Zn₂Ti₍₁₋ₓ₎FeₓO₄ refers to a mathematical representation of a chemical formula in which Ti atoms (1-x) are optionally substituted with Fe atoms (x).

The doped or undoped zinc titanate crystals are a composite material, having properties which differ from those individually characterizing their starting compounds. One or more crystals, of the same or different general chemical formula, may form particles or nanoparticles as described below.

The zinc titanate crystals can be synthesized using different ratios of zinc oxide (ZnO; also referred to as zinc(II) oxide) and titanium dioxide (TiO_{2;} often referred to as titanate or titanium oxide) by a variety of methods readily known to the person skilled in the art of preparing such composite materials.

In the event that iron atoms (as available for instance from iron(III) oxide or ferric oxide (Fe₂O₃)) optionally substitutes atoms of the composite material, typically titanium, the so-called "doped" crystal is formed. In such case, in the formula Zn₂Ti₍₁₋ₓ₎FeₓO₄, x equals a number greater than 0. In some embodiments, the crystal is undoped. In such a case, x equals 0.

In some embodiments, x is between 0.005 and 0.1, optionally between 0.025 and 0.05, having values such as 0.025, 0.03, 0.035, 0.04, 0.045 or 0.05.

In some embodiments, x equals 0.025. In some embodiments, x equals 0.05.

In the following, a zinc titanate crystal wherein x equals zero can also be referred to as an undoped zinc titanate crystal, while a zinc titanate crystal wherein x is greater than zero can also be referred to as a doped or an Fe-doped zinc titanate crystal.

The compositions described herein are for use in both living subjects and inanimate objects (*e.g.,* UV protective coating of articles routinely exposed to UV radiation).

Therefore, some embodiments of the present disclosure relates to compositions providing protection against ultraviolet radiation (*i.e.* UV protective compositions), and more particularly, to UV protective compositions comprising zinc titanate crystals, optionally doped by iron atoms, as an ultraviolet-absorbing agent.

In some embodiments, the doped or undoped zinc titanate crystals are in the form of nanoparticles consisting of one or more crystals, at least 50% of the total number of the nanoparticles having at least one dimension of up to about 200 nm, or up to about 150 nm, or up to about 100 nm. Such as at least 55, 60, 65, 70, 75, 80, 85, 90, 95, 96, 97, 98 or 99% of the nanoparticles have at least one dimension of up to about 50, 60, 70, 80, 90, 100, 110, 120, 130, 140, 150, 160, 170, 180, 190 or 200 nm. In some embodiments, at least 90% of the total number of the nanoparticles of doped or undoped zinc titanate crystals has at least one dimension of up to about 200 nm, or up to about 150 nm, or up to about 100 nm. In some embodiments, the nanoparticles consist of crystals having the same chemical formula.

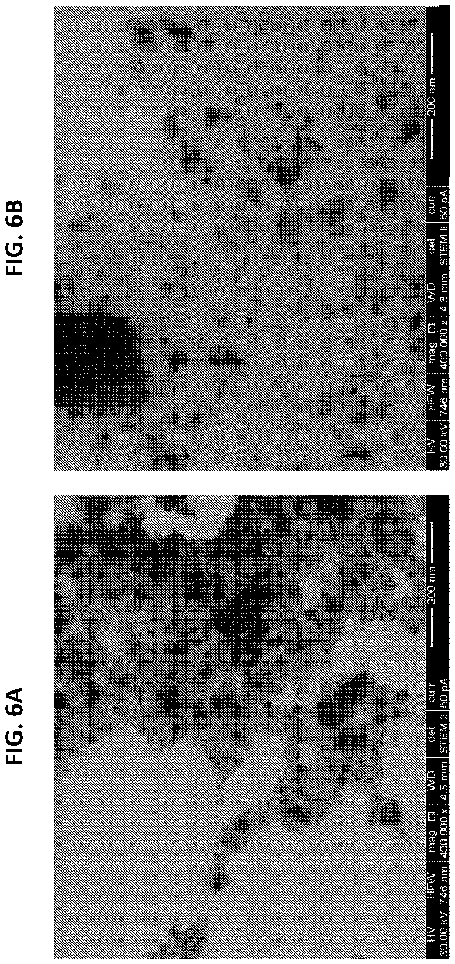

As employed herein with respect to a nanoparticle, "having at least one dimension" refers, in some embodiments, to the longest dimension of the particle, which can typically be approximated to a diameter, the crystals of zinc titanate, for instance, having roughly a globular shape, see for example Figure 6.

In some embodiments, the doped or undoped zinc titanate crystals are in the form of nanoparticles consisting of one or more crystals, at least 50% of the total volume of the nanoparticles having at least one dimension of up to about 200 nm, or up to about 150 nm, or up to about 100 nm. Such as at least 55, 60, 65, 70, 75, 80, 85, 90, 95, 96, 97, 98, or 99% of the nanoparticles have at least one dimension of up to about 50, 60, 70, 80, 90, 100, 110, 120, 130, 140, 150, 160, 170, 180, 190 or 200 nm. In some embodiments, at least 90% of the total volume of the nanoparticles of doped or undoped zinc titanate crystals has at least one dimension of up to about 200 nm, or up to about 150 nm, or up to about 100 nm. In some embodiments, the nanoparticles consist of crystals having the same chemical formula

In some embodiments, at least 55%, at least 60%, at least 65%, at least 70%, at least 80%, or at least 85% of the total number or total volume of nanoparticles of the doped or undoped zinc titanate crystals has at least one dimension of up to about 200 nm, in some embodiments up to about 150 nm and even of up to about 100 nm.

In some embodiments, at least 50%, at least 55%, at least 60%, at least 65%, at least 70%, at least 80%, at least 85%, at least 90%, at least 95%, at least 97.5%, or at least 99% of the total number or total volume of nanoparticles present in the composition has a hydrodynamic diameter of up to about 200 nm, or up to 150 nm, or even up to about 100 nm.

In some embodiments, the nanoparticles of doped or undoped zinc titanate are present in the composition dispersed in a polymer matrix. In particular embodiments the nanoparticles of the composite UV-absorbing agent are dispersed in the polymer matrix in presence of a dispersant, the polymer matrix being in an oil-based carrier.

In some embodiments, the UV-protective composition disclosed herein is generally devoid and/or generally free of an organic ultraviolet-absorbing agent, the composition optionally containing less than 5 wt.%, less than 4 wt.%, less than 3 wt.%, less than 2 wt.%, less than 1 wt.%, less than 0.5 wt.%, less than 0.1 wt.% or less than 0.05 wt.% organic ultraviolet-absorbing agent(s).

In some embodiments, the UV-protective composition disclosed herein is generally devoid and/or generally free of an additional inorganic ultraviolet-absorbing agent, the composition optionally containing less than 5 wt.%, less than 4 wt.%, less than 3 wt.%, less than 2 wt.%, less than 1 wt.%, less than 0.5 wt.%, less than 0.1 wt.% or less than 0.05 wt.% additional inorganic ultraviolet-absorbing agent(s).

In some embodiments, the doped or undoped zinc titanate crystals, optionally in the form of nanoparticles, constitute the only ultraviolet-absorbing agents in the UV-protective composition disclosed herein.

In some embodiments, the doped or undoped zinc titanate crystals, optionally in the form of nanoparticles, are present at a concentration in the range of from about 0.001% to about 40% (w/w or wt.%) of the UV-protective composition disclosed herein. In some embodiments, nanoparticles of zinc titanate crystals constitute about 0.01, 0.1, 1, 2, 3, 4, 5, 10, 15, 20, 25, 30 or 35% (w/w) of the UV-protective composition.

In some embodiments, the UV-protective composition further comprises silver particles.

In some embodiments, the silver particles comprise silver nanoparticles having at least one dimension of up to about 50 nm. In some embodiments, the silver nanoparticles have at least one dimension (*e.g.,* a diameter) up to about 10, 20, 30 or 40 nm.

In some embodiments, at least 90%, at least 95%, at least 97.5% or at least 99% of the number of silver nanoparticles present in the composition has at least one dimension of up to about 50 nm.

In some embodiments, at least 90%, at least 95%, at least 97.5% or at least 99% of the volume of silver nanoparticles present in the composition have at least one dimension of up to about 50 nm. In some embodiments, wherein the composition comprises silver nanoparticles, the composition is devoid of an additional ultraviolet-absorbing agent.

In some embodiments, the silver particles are present in the composition at a concentration in the range of from about 0.01% to about 10% (w/w) of the total composition. In some embodiments, the silver particles constitute about 0.1, 1, 2, 3, 4, 5, 6, 7, 8, or 9% (w/w) of the total composition.

In some embodiments, the composition further comprises one or more of a carrier, an excipient, an additive and combinations thereof. Carriers, excipients and additives being cosmetically acceptable are preferred for use in living subjects, but may not be required for use on the surfaces of inanimate objects. In one embodiment the carrier, excipient or additive is cosmetically acceptable.

In some embodiments, the UV-protective composition is in a form selected from the group consisting of an aerosol, a cream, an emulsion, a gel, a lotion, a mousse, a paste, a liquid coat, a film, a powder and a spray.

In some embodiments, the UV-protective composition is formulated as one or more of the following: (a) a skin-care composition for application to human or non-human animal skin; (b) a hair-care composition for application to human or non-human animal hair; or (c) a coating composition for application to an inanimate surface.

In a further aspect, embodiments of the present disclosure provide use of afore-described doped or undoped zinc titanate crystals, optionally in the form of nanoparticles, for the preparation of a composition for protecting a target surface, such as a surface of a living subject and/or an inanimate object, against an effect of UV radiation (*e.g.,* a harmful effect such as a chemical modification of the exposed surface). The compositions, comprising an efficacious amount of zinc titanate crystals, can be formulated as suitable for application upon the intended surfaces, such preparations being known to persons skilled in the relevant formulations.

In one embodiment, an effect of UV radiation refers to a harmful effect of UV radiation such as, by way of example, a chemical modification of the exposed surface which includes, but is not limited to: bleaching, color alteration, burning, ageing or fragilization (*e.g.,* of the hair).

According to one embodiment, there is provided a composition as described herein, for use in protecting a subject against an effect of UV radiation

According to one embodiment, there is provided a composition as described herein, for use in protecting the skin of a subject against an effect of UV radiation. In some such embodiments the composition is in the form of a topical composition. In such embodiments, the composition can be in any form suitable to skin-care products, such as facial-care products, make-up products, body-care products, hand-care products and/or foot-care products. Such skin-care products can be applied to the skin of a subject by any conventional method and/or for any duration of time that need not be detailed herein.

According to a further embodiment, there is provided a composition as described herein, for use in protecting the hair of a subject against an effect of UV radiation. In some such embodiments, the composition is in the form of a hair-care product, such as a hair-care product selected from the group consisting of a shampoo, a conditioner, a hair spray and a hair mask. Such hair-care products can be applied to the hair of a subject by any conventional method and/or for any duration of time that need not be detailed herein.

In some embodiments of a use of the composition, the subject is a human subject. In alternative embodiments of a use of the composition, the subject is a non-human animal.

In some embodiments of the use of the composition, the target surface is a surface of an inanimate object, such as, for example, an object, or a material. In some such embodiments, the composition is in the form of a coating, including liquid coatings, such as a varnish, a lacquer or an emulsion, and non-liquid coatings, such as a paste, a gel, a film, a powder or a mousse. Though UV-protective compositions applicable to the surfaces of inanimate objects are herein referred to as "coatings", it will be readily understood that such compositions may also permeate, impregnate or be otherwise embedded at least to some extent within the surfaces of the objects being protected. Such coating products can be applied to the surface of an inanimate object by any conventional method that need not be detailed herein.

In some embodiments, protecting against ultraviolet radiation comprises protecting against an effect of ultraviolet A radiation, ultraviolet B radiation or ultraviolet A and ultraviolet B radiation.

In some embodiments, the composition has a critical wavelength of at least 370 nm, such as 371 nm, 372 nm, 373 nm, 374 nm, 375 nm, 376 nm, 377 nm, 378 nm, 379 nm, 380 nm, 381 nm, 382 nm, 383 nm, 384 nm, 385 nm, 386 nm, 387 nm, 388 nm, 389 nm, 390 nm, 391 nm, 392 nm, or greater than 392 nm.

In some embodiments, the area under the curve (AUC) formed by the UV-absorption of the doped or undoped zinc titanate crystals as a function of wavelength in the range of 280 nm to 400 nm (AUC₂₈₀₋₄₀₀) is at least 75%, at least 85% or at least 95% of the AUC formed by the same doped or undoped zinc titanate crystals at the same concentration in the range of 280 nm to 700 nm (AUC₂₈₀₋₇₀₀).

According to a further aspect of some embodiments of the disclosure, there is provided a method of manufacturing a UV-protective composition, comprising combining doped or undoped zinc titanate crystals, as an ultraviolet-absorbing agent, with other ingredients in proportions and in a manner suitable to make a UV-protective composition as described herein. In some embodiments, the UV-protective composition is manufactured and formulated as a sunscreen composition for application to skin or hair of a human or non-human living subject. In some embodiments, the composition is manufactured and formulated as a composition for application to a surface of an inanimate object.

There is also provided, in accordance with an embodiment of the invention, a method of protecting a surface from UV radiation, which comprises applying to a surface in need of such protection a UV-protective composition as described herein in an amount sufficient to achieve such protection. In some embodiments, the surface is human skin. In some embodiments, the surface is non-human skin, *i.e.* animal skin. In some embodiments, the surface is hair. In some embodiments, the hair is human hair. In some embodiments, the hair is non-human hair, *i.e.* animal hair. In some embodiments, the surface is a surface of an inanimate object.

As used herein, the term "nanoparticles" refers to particles of any suitable shape, which may consist of one or more crystals as herein disclosed, wherein the size of at least one dimension is 200 nm or less, such as 10, 20, 30, 40, 50, 60, 70, 80, 90, 100, 110, 120, 130, 140, 150, 160 170, 180 or 190 nm or less, hereinafter also referred to as the smallest dimension, and wherein a greatest size in a different dimension of the particles, also termed a greatest dimension, is of no more than about 500 nm, such as no more than about 490, 480, 470, 460, 450, 440, 430, 420, 410, 400, 390, 380, 370, 360, 350, 340, 330, 320, 300, 290, 280, 270, 260, 250, 240, 230, 220 or 210 nm.

For example, in some embodiments where the particles have a flake-like shape, the smallest dimension of the nanoparticles can be their thickness which can be of up to about 200 nm, while their length can be of no more than about 500 nm.

For example, in some embodiments where the particles have a rod-like shape, their cross section along their longitudinal axis could be approximated to ellipsoids having at least their minor axis constituting a smallest dimension of no more than about 200 nm and the length of the rods being no more than about 500 nm.

For example, in some embodiments where the particles have a sphere-like shape that could be approximated by three diameters one for each of the X-, Y- and Z-direction, at least one of the three diameters is not more than about 200 nm and a greatest of the three diameters can be no more than about 500 nm.

In some embodiments, the smallest dimension of the nanoparticles is not more than about 180 nm, not more than about 160 nm, not more than about 140 nm, not more than about 120 nm, or even not more than about 100 nm.

In some embodiments, the smallest dimension of the nanoparticles is at least about 10 nm, at least about 15 nm or at least about 20 nm.

In some embodiments, the greatest dimension of the nanoparticles is not more than about 400 nm, not more than about 300 nm, not more than about 200 nm, or even not more than about 150 nm.

In some embodiments, the nanoparticles of doped or undoped zinc titanate crystals and/or the compositions including the doped or undoped zinc titanate crystals disclosed herein are substantially invisible to the human eye, in particular when applied to a subject.

In some embodiments, the compositions are visible to the human eye when applied to a subject. In some such embodiments, iron doped zinc titanate crystals provide a pale reddish colour that is beneficial in the preparation of a product in which such colour is desirable, *e.g.* a make-up product such as a blusher, or a tinted coating for application to a surface of an inanimate object.

In some embodiments, the size of the particles is determined by microscopy techniques, as known in the art.

In some embodiments, the size of the particles is determined by Dynamic Light Scattering (DLS). In DLS techniques the particles are approximated to spheres of equivalent behavior and the size can be provided in term of hydrodynamic diameter. DLS also allows more readily assessing the size distribution of a population of particles.

Distribution results can be expressed in terms of the hydrodynamic diameter for a given percentage of the cumulative particle size distribution, either in terms of numbers of particles or volumes, and are typically provided for 10%, 50% and 90% of the cumulative particle size distribution. For instance, D50 refers to the maximum hydrodynamic diameter below which 50% of the sample volume or number of particles, as the case may be, exists and is interchangeably termed the median diameter per volume (Dv50) or per number (D_{N}50), respectively.

In some embodiments, the nanoparticles of the disclosure have a cumulative particle size distribution of D90 of 200 nm or less, or a D95 of 200 nm or less, or a D97.5 of 200 nm or less or a D99 of 200 nm or less, *i.e.* 90%, 95%, 97.5% or 99% of the sample volume or number of particles respectively, have a hydrodynamic diameter of no greater than 200 nm.

In some embodiments, the cumulative particle size distribution of the population of nanoparticles is assessed in term of number of particles (denoted D_{N}) or in term of volume of the sample (denoted Dv) comprising particles having a given hydrodynamic diameter.

Any hydrodynamic diameter having a cumulative particle size distribution of 90% or 95% or 97.5% or 99% of the particles population, whether in terms of number of particles or volume of sample, may be referred to hereinafter as the "maximum diameter", *i.e.* the maximum hydrodynamic diameter of particles present in the population at the respective cumulative size distribution.

It is to be understood that the term "maximum diameter" is not intended to limit the scope of the present teachings to nanoparticles having a perfect spherical shape. This term as used herein encompasses any representative dimension of the particles at cumulative particle size distribution of at least 90%, *e.g.,* 90%, 95%, 97.5% or 99%, or any other intermediate value, of the distribution of the population.

As used herein, the terms "ultraviolet-protective agent" or "ultraviolet-protecting agent" refer to agents that absorb and/or reflect and/or scatter at least some of the UV radiation on surfaces exposed to sunlight or any other UV source, and thus reduce the effect of UV radiation on the surface. The surface may be the skin and/or hair of a subject, such as a human subject. The surface may also be the surface (*e.g.,* an exterior face) of an inanimate object.

In another aspect, embodiments of the present disclosure provide a method for the preparation of afore-described compositions.

Some known UV protective compositions block both UVA and UVB radiation by use of a combination of different UV-protecting agents, each of which blocks radiation over a limited range of the UV spectrum.

As used herein, the term "broad-spectrum UV absorption" with regard to an ultraviolet-absorbing agent refers to an ultraviolet-absorbing agent that absorbs both UVA and UVB radiation. In some embodiments, the breadth of UV absorption may be measured according to the Critical Wavelength Method, wherein an ultraviolet-absorbing agent is considered to provide broad spectrum absorption when the critical wavelength is greater than 370 nm, and unless otherwise noted, in the present disclosure the term "broad-spectrum UV absorption" as used herein is determined on the basis of the critical wavelength.

As used herein, the term "critical wavelength" is defined as the wavelength at which the area under the absorbance spectrum from 290 nm to the critical wavelength constitutes 90% of the integral of the absorbance spectrum in the range from 290 nm to 400 nm.

In some instances, noted as such herein, the term "broad-spectrum UV absorption" with regard to an ultraviolet-absorbing agent refers to the situation in which the area under the curve (AUC) formed by the UV-absorption of the agent as a function of wavelength in the range of 280 nm to 400 nm (AUC₂₈₀₋₄₀₀) is at least 75% of the AUC formed by the same agent at the same concentration in the range of 280 nm to 700 nm (AUC₂₈₀₋₇₀₀). Similarly, where noted as such herein, the terms "broader-spectrum UV absorption" and "broadest spectrum UV absorption" with respect to a UV-absorbing agent refer respectively to the situation in which the area under the curve (AUC) formed by the absorption of the agent as a function of wavelength in the range of 280 nm to 400 nm (AUC₂₈₀₋₄₀₀) is at least 85% or 95% of the AUC formed by the same agent at the same concentration in the range of 280 nm to 700 nm (AUC₂₈₀₋₇₀₀).

As used herein, the term "ultraviolet-absorbing agent" refers to an agent which, when present in a composition at up to 50% (w/w) of the total composition, provides at least 50% absorption of ultraviolet light in the wavelength range of from 290 nm to 400 nm.

As used herein, the terms "generally devoid of an organic ultraviolet-absorbing agent", "considerably devoid of an organic ultraviolet-absorbing agent", "significantly devoid of an organic ultraviolet-absorbing agent", "substantially devoid of an organic ultraviolet-absorbing agent", "essentially devoid of an organic ultraviolet-absorbing agent", "substantively devoid of an organic ultraviolet-absorbing agent" and "devoid of an organic ultraviolet-absorbing agent" refer respectively to a composition in which a UV-absorbing organic material, if any, is present in the composition at a concentration which provides absorption of not more than 20%, not more than 15%, not more than 10%, not more than 5%, not more than 2%, not more than 1% or not more than 0.5% of ultraviolet light in the wavelength range of from 290 nm to 400 nm.

As used herein, the term "generally devoid of an additional ultraviolet-absorbing agent", "considerably devoid of an additional ultraviolet-absorbing agent", "significantly devoid of an additional ultraviolet-absorbing agent", "substantially devoid of an additional ultraviolet-absorbing agent", "essentially devoid of an additional ultraviolet-absorbing agent", "substantively devoid of an additional ultraviolet-absorbing agent" and "devoid of an additional ultraviolet-absorbing agent" refer respectively to a composition which is devoid of any UV-absorbing material other than that specifically disclosed as being present in the composition at a concentration, which, if included in the composition, provides absorption of not more than 20%, not more than 15%, not more than 10%, not more than 5%, not more than 2%, not more than 1% or not more than 0.5% of ultraviolet light in the wavelength range of from 290 nm to 400 nm. In some embodiments, the additional ultraviolet-absorbing agent of which the composition is at least generally devoid is an inorganic UV-absorbing agent.

According to an aspect of some embodiments, the present disclosure relates to compositions providing protection against ultraviolet radiation, and more particularly, to UV protective compositions comprising a matrix comprising a polymer and an oil, and doped or undoped zinc titanate crystals and a dispersant, wherein the zinc titanate crystals are dispersed in the matrix. Advantageously, the dispersed zinc titanate crystals do not substantially migrate out of the polymer matrix. In such case, the zinc titanate crystals may also be said to be immobilised in the matrix, also referred to as the polymer matrix or the swelled polymer matrix.

According to an aspect of some embodiments of the disclosure, there is provided a matrix comprising a polymer and an oil; and doped or undoped zinc titanate crystals and a dispersant, dispersed in the matrix.

In some embodiments, the UV-protective composition provides protection against UV radiation selected from the group consisting of a UVA-radiation and a UVB-radiation. In some embodiments, the UV-protective composition provides UVA and UVB protective activity.

In some embodiments, the doped or undoped zinc titanate crystals are present in the matrix at a concentration of from about 0.1 to about 60% (w/w) of the polymer, such as about 1, 2, 3, 4, 5, 10, 15, 20, 25, 30, 35, 40, 45, 50 or 55% (w/w) of the polymer, or from about 3 to about 40%, optionally at a concentration of about 5 to 20% (w/w) of the polymer.

In some embodiments, the doped or undoped zinc titanate crystals are present in the matrix at a concentration of from about 0.01 to about 8% (v/v or v.%) of the polymer, such as about 0.1, 1, 2, 3, 4, 5, 6 or 7% (v/v) of the polymer, or from about 0.4 to about 5% (v/v), optionally at a concentration of about 0.6 to about 3% (v/v) of the polymer.

In some embodiments, the doped or undoped zinc titanate crystals are present in the matrix at a concentration of from about 1 to about 10% (w/w) or from about 0.1 to about 10% (v/v) of the total composition, such as about 0.1, 1, 2, 3, 4, 5, 6, 7, 8 or 9% (w/w) or (v/v) of the composition, optionally at a concentration of about 4% (w/w) or 0.5% (v/v) of the composition.

In some embodiments, the oil is present at a concentration of from about 10 to about 50% (w/w) of the polymer of the matrix, such as about 15, 20, 25, 30, 35, 40, or 45% (w/w) of the matrix, or from about 5 to about 50% (v/v) of said matrix, such as about 5, 10, 15, 20, 25, 30, 35, 40, or 45% (v/v) of the matrix optionally at a concentration of about 30% (w/w) or about 20% (v/v) of the matrix.

In some embodiments, the oil of the polymer matrix is selected from the group consisting of mineral oil, natural oil, vegetal oil, synthetic oil, and combinations thereof.

In some embodiments, the polymer of the matrix is an oil-swellable thermoplastic homo- or co- polymer, optionally clear, transparent and/or colorless.

In some preferred embodiments, the polymers suitable for the matrix are functionalized polymers or copolymers comprising particle-affinic functional group and non-affinic monomer units. For instance, the functional groups may be acidic monomers, whereas the non-affinic groups can be ethylene. In some embodiments, the polymer comprises at least one ethylene-acrylic (EAA) polymer, ethylene-methacrylic (EMMA) polymer, ethyl vinyl acetate (EVA) polymer, and combinations thereof.

In some embodiments, the polymer of the matrix comprises at least one ethylene-acrylic polymer, optionally wherein the ethylene-acrylic polymer comprises from about 5 to about 30% (w/w) acrylic monomer, such as about 10, 15, 20, or 25% (w/w) acrylic monomer. In some embodiments, the ethylene-acrylic polymer is selected from the group consisting of ethylene-methacrylic acid copolymer and ethylene-acrylic acid copolymer.

In some embodiments, the polymer of the matrix, which can be a copolymer or a combination thereof, have at least one of a softening point and a melting point not exceeding 200°C, said softening point or melting point optionally being of at least 60°C.

The oil and the polymer of the polymer matrix, or a combination of oils and/or a combination of polymers forming such a matrix, are selected and adapted to be compatible one with the other. In other words the oil(s) can swell the polymer(s) and the polymer(s) can be swelled by the oil(s). Swelling (and grammatical variants) refers to the ability of the oil to penetrate a polymeric network formed by the polymer (the matrix), resulting, among other things, in an increase in the weight of the matrix, and typically additionally in an expansion of its volume.

In some embodiments, the matrix is present in the form of matrix elements, at least 50% of the number of matrix elements having at least one dimension of up to about 50 µm, at most 25 µm, at most 10 µm or at most 5 µm.

In some embodiments, the matrix elements of the polymer matrix (*e.g.,* comprising a thermoplastic polymer swelled with an oil and nanoparticles of doped or undoped zinc titanate crystals dispersed and embedded therein with a dispersant) are matrix flakes, wherein each flake of the swelled polymer matrix flakes has a flake length (Lf), a flake width (Wf), and a flake thickness (Tf), the matrix flakes having a dimensionless flake aspect ratio (Rf) defined by: $Rf = \left(Lf\cdot Wf\right) / {\left(Tf\right)}^{2}$ wherein, with respect to a representative group of the swelled polymer matrix flakes, an average Rf is at least 5.

In some embodiments, at least one of the flake length (Lf) and the flake width (Wf) of the matrix flakes is at most 50 µm, at most 25 µm, at most 10 µm, or at most 5 µm.

In some embodiments, the flake thickness (Tf) of the matrix flakes is at most 1000 nm, at most 900 nm, at most 750 nm, at most 650 nm, at most 600 nm, at most 550 nm, at most 500 nm, at most 450 nm, at most 400 nm, at most 350 nm, at most 300 nm, or at most 250 nm.

In some embodiments, flake aspect ratio (Rf) of the matrix flakes is within a range of from about 5 to about 2000, from about 10 to about 1000, from about 12 to about 500, from about 12 to about 200, or from about 15 to about 100.

In some embodiments, the representative group is disposed in an instrumental field of view containing at least 10 of the matrix flakes or swelled polymer matrix flakes, and optionally hundreds of nanoparticles of doped or undoped zinc titanate crystals.

In some embodiments, at least 50%, at least 60%, at least 75%, or at least 90% of the nanoparticles embedded in the matrix elements or matrix flakes have a cumulative particle size (D50, D60, D75, and D90, accordingly) of at most 100 nm, at most 90 nm, at most 80 nm, at most 70 nm, or at most 60 nm. The cumulative particle size can be determined in terms of percent number of nanoparticles in the population of the plurality of particles or in terms of percent volume. Thus, in some embodiments, the nanoparticles of doped or undoped zinc titanate crystals embedded in the matrix flakes can be characterized by a D_{N}50 of at most 100 nm (up to a D_{N}90 of at most 60 nm) or by a Dv50 of at most 100 nm (up to a Dv90 of at most 60 nm).

In some embodiments, the dispersant adapted to disperse the nanoparticles of doped or undoped zinc titanate crystals within the polymer matrix has a hydrophilic-lipophilic balance (HLB) value of at most 9, at most 6, at most 4, or at most 3.

Aspects and embodiments of the disclosure are described in the specification herein below and in the appended claims.

Unless otherwise defined, all technical and scientific terms used herein have the same meaning as commonly understood by one of ordinary skill in the art to which the particular teachings pertain. In case of conflict, the specification, including definitions, will take precedence.

As used herein, the terms "comprising", "including", "having" and grammatical variants thereof are to be taken as specifying the stated features, integers, steps or components, but do not preclude the addition of one or more additional features, integers, steps, components or groups thereof. These terms encompass the terms "consisting of' and "consisting essentially of'.

As used herein, the indefinite articles "a" and "an" and the singular form "the" include plural references and mean "at least one" or "one or more" unless the context clearly dictates otherwise.

Unless otherwise stated, the use of the expression "and/or" between the last two members of a list of options for selection indicates that a selection of one or more of the listed options is appropriate and may be made.

In the discussion, unless otherwise stated, adjectives such as "substantially" and "about" that modify a condition or relationship characteristic of a feature or features of an embodiment of the present technology, are to be understood to mean that the condition or characteristic is defined within tolerances that are acceptable for operation of the embodiment for an application for which it is intended, or within variations expected from the measurement being performed and/or from the measuring instrument being used. In particular, when a numerical value is preceded by the term "about", the term "about" is intended to indicate +/-10%, or +/-5%, or +/-2% of the mentioned value and in some instances the precise value.

Additional objects, features and advantages of the present teachings, and aspects of embodiments of the invention, will be set forth in the detailed description which follows, and in part will be readily apparent to those skilled in the art from the description or recognized by practicing embodiments of the invention as described in the written description and claims hereof, as well as the appended drawings. Various features and sub-combinations of embodiments of the present disclosure may be employed without reference to other features and sub-combinations.

It is to be understood that both the foregoing general description and the following detailed description, including the materials, methods and examples, are merely exemplary, and are intended to provide an overview or framework to understanding the nature and character of the invention as it is claimed, and are not intended to be necessarily limiting.

### BRIEF DESCRIPTION OF THE DRAWINGS

Some embodiments of the invention are described herein with reference to the accompanying figures. The description, together with the figures, makes apparent to a person having ordinary skill in the art how some embodiments of the disclosure may be practiced. The figures are for the purpose of illustrative discussion and no attempt is made to show structural details of an embodiment in more detail than is necessary for a fundamental understanding of the disclosure. For the sake of clarity, some objects depicted in the figures are not to scale.

In the Figures:
Figure 1 is a line graph showing powder absorbance of Fe-doped and undoped zinc titanate powder, prepared according to present teachings, as compared to undoped zinc oxide powder and presintered zinc titanate as reference.
Figure 2 is a plot showing the powder X ray diffraction (PXRD) diffractogram of Fe-doped and undoped zinc titanate crystals prepared according to the present teachings.
Figure 3 is a line graph showing Particle Size Distribution (PSD) of particles of Fe-doped and undoped zinc titanate powder after milling according to present teachings, expressed as number percentage, as compared to zinc oxide as reference.
Figure 4 is a line graph showing absorbance of aqueous suspensions comprising different concentrations of nanoparticles of Fe-doped zinc titanate crystals, prepared according to present teachings, as compared to the same respective concentrations of undoped zinc titanate as reference.
Figure 5 is a line graph showing absorbance of aqueous suspensions comprising a same concentration of nanoparticles of zinc titanate crystals at various levels of Fe-doping prepared according to present teachings, as compared to undoped zinc titanate crystals, undoped zinc oxide and a commercially available sunscreen as reference.
Figure 6 is a high resolution Scanning Electron Microscope (HR-SEM) image of nanoparticles of zinc titanate crystals prepared according to present teachings, panel A showing nanoparticles of undoped zinc titanate crystals and panel B showing nanoparticles of Fe-doped zinc titanate crystals.

### DETAILED DESCRIPTION

The present disclosure, in at least some embodiments, provides compositions for protection against ultraviolet radiation, uses of such compositions and methods of making such compositions.

The UV protective compositions disclosed herein comprise one or more zinc titanate crystals each independently having the chemical formula Zn₂Ti₍₁₋ₓ₎FeₓO₄, wherein x is between 0 and 0.1, which when present as large particles (*e.g.,* dimensions in each of the X-, Y- and Z-directions being greater than 200 nanometers (nm), resulting for instance in a hydrodynamic diameter of more than 200 nm as measured by DLS) may effectively absorb radiation having wavelengths of greater than about 400 nm. Accordingly, compositions comprising such large particles of zinc titanate crystals, whether or not further substituted (doped) by iron atoms, may provide protection against ultraviolet radiation having wavelengths up to at least 400 nm.

However, in the case in which the UV-protective composition is a sunscreen composition which comprises doped or undoped zinc titanate crystals, but which also contains particles that absorb light at wavelengths in the range of 400-800 nm, the sunscreen will be visible on the end-user because of the absorption in the visible range (> 400 nm).

It has surprisingly been found by the present Inventors that, although reduction of particle size of known inorganic UV-absorbing agents to dimensions below 1 micrometer (µm), typically below 100 nm (for instance, reduction to nanometric dimensions) is known to significantly reduce the maximum wavelength of light, including UV light, which is effectively absorbed by the particles, UV protective compositions according to the present teachings comprising particles of doped or undoped zinc titanate crystals milled to nanoparticle size still provide substantial absorption of UV radiation of wavelength from 280 nm (or even shorter wavelength) up to about 400 nm, thus providing broad-spectrum protection against both UVA and UVB radiation, even in the absence of additional ultraviolet-absorbing agents.

Thus, in some embodiments, UV protective compositions disclosed herein, such as sunscreen compositions, comprise doped or undoped zinc titanate in the form of particles, comprising one or more said crystals, wherein at least 90% of the particles are nanoparticles, such as at least 91, 92, 93, 94, 95, 96, 97, 98, or 99% of the particles are nanoparticles. In some embodiments, at least 95%, or at least 97.5% or at least 99% of the particles, in terms of number or volume of particles, are nanoparticles. In some embodiments, at least one dimension of the zinc titanate crystal nanoparticles is expressed in terms of the hydrodynamic diameter as measured by DLS techniques.

In some embodiments, the cumulative particle size distribution in a sample is assessed in terms of the number of particles in the sample (denoted D_{N}). In some embodiments, the cumulative particle size distribution in a sample is assessed in terms of the volume of particles in the sample (denoted D_{V}).

In some embodiments, the maximum diameter of the nanoparticles is assessed for population distribution measured in terms of number of particles and percentage thereof. In some embodiments, the maximum diameter of the nanoparticles is assessed for population distribution measured in terms of sample volume of particles and percentage thereof.

Dimensions of particles can also be assessed (or confirmed) by microscopy (*e.g.,* light microscopy, confocal microscopy, SEM, STEM, *etc*.). Such techniques are deemed more suitable than DLS for particles (such as matrix flakes) having non-globular shapes. The particles may be characterized by an aspect ratio, *e.g.,* a dimensionless ratio between the smallest dimension of the particle and the longest dimension or equivalent diameter in the largest plane orthogonal to the smallest dimension, as relevant to their shape. The equivalent diameter (Deq) is defined by the arithmetical average between the longest and shortest dimensions of that largest orthogonal plane. Particles having an almost spherical shape are characterized by an aspect ratio of approximately 1:1, whereas flake-like particles, such as matrix flakes, can have an aspect ratio of up to 1:100, or more.

As further detailed herein-below, nanoparticles of doped or undoped zinc titanate crystals can in some embodiments be immobilised within a polymer matrix. The matrix can form distinct elements, which may assume a variety of shapes. For topical application, a platelet shape is deemed particularly suitable. Such matrix flakes can be characterized by a flake length (Lf, the longest dimension in the plane of the flake), a flake width (Wf, the largest dimension in the plane of the flake, such width being orthogonal to the length), and a flake thickness (Tf, the largest thickness being measured orthogonally to the plane in which the length and width of the flake are defined). Lf, Wf and Tf can be further used to calculate an aspect ratio (*e.g.,* Rf as below defined) of a matrix flake.

Such characteristic dimensions can be assessed on a number of representative particles, or a group of representative particles, that may accurately characterize the population (*e.g.,* by diameter, longest dimension, thickness, aspect ratio and like characterizing measures of the particles). It will be appreciated that a more statistical approach may be desired for such assessments. When using microscopy for particle size characterization, a field of view of the image-capturing instrument (*e.g.,* light microscope, *etc*.) is analyzed in its entirety. Typically, the magnification is adjusted such that at least 5 particles, at least 10 particles, at least 20 particles, or at least 50 particles are disposed within a single field of view. Naturally, the field of view should be a representative field of view as assessed by one skilled in the art of microscopic analysis. The average value characterizing such a group of particles in such a field of view is obtained by volume averaging. In such case, D_{V}50 = ∑[(Deq(*m*))³/*m*]^{1/3}, wherein *m* represents the number of particles in the field of view and the summation is performed over all *m* particles. As mentioned, when such methods are the technique of choice for the scale of the particles to be studied or in view of their shape, such measurements can be referred to as D50.

In some embodiments, the doped or undoped nanoparticles of zinc titanate crystals are substantially invisible to the human eye, in particular when applied to the skin or hair of a subject, or if desired when applied to an inanimate surface, due to their small size.

In some embodiments, the doped or undoped nanoparticles of zinc titanate crystals are blended into a coloured composition and need not be substantially transparent and/or invisible, for instance when used in a make-up product, such as a foundation, which is slightly tinted when applied to the skin of a subject, or when used in a stain or paint applicable to inanimate surfaces.

According to some embodiments of the disclosure, there is provided a UV protective composition comprising undoped zinc titanate crystals.

According to some embodiments of the disclosure, there is provided a UV protective composition comprising Fe-doped zinc titanate crystals, the level of doping by iron atoms being such that the Ti:Fe molar ratio can be between 50:1 and 2:1, such as 49:1, 48:1, 47:1, 46:1, 45:1, 44:1, 43:1, 42:1, 41:1, 40:1, 39:1, 38:1, 37:1, 36:1, 35:1, 34:1, 33:1, 32:1, 31:1, 30:1, 29:1, 28:1, 27:1, 26: 1, 25:1, 24:1, 23:1, 22:1, 21:1, 20:1, 19:1, 18:1, 17:1, 16:1, 15:1, 14:1, 13:1, 12:1, 11:1, 10:1, 9:1, 8:1, 7:1, 6:1, 5:1, 4:1 or 3:1, in particular between Ti:Fe 0.975:0.025 (39:1) and 0.95:0.05 (19:1).

According to a further aspect of some embodiments of the disclosure, there is provided a UV protective composition comprising doped or undoped zinc titanate crystals for use in protecting the skin of a subject, such as a human subject, against ultraviolet radiation, in some embodiments providing broad-spectrum protection against both ultraviolet A and ultraviolet B radiation.

According to a further aspect of some embodiments of the disclosure, there is provided a UV protective composition comprising doped or undoped zinc titanate crystals for use in protecting the hair of a subject, such as a human subject, against ultraviolet radiation, in some embodiments against both ultraviolet A and ultraviolet B radiation.

According to a further aspect of some embodiments of the disclosure, there is provided a method of protecting the skin of a subject against ultraviolet radiation, the method comprising applying to the skin of the subject an efficacious amount of a UV protective composition comprising doped or zinc titanate crystals. In some such embodiments, the UV-protective composition can be in the form of a skin-care product suitable for skin application and/or at least temporary retention thereupon.

According to a further aspect of some embodiments of the disclosure, there is provided a method of protecting the hair of a subject against ultraviolet radiation, the method comprising applying to the hair of the subject an efficacious amount of a UV protective composition comprising doped or undoped zinc titanate crystals. In some such embodiments, the UV-protective composition can be in the form of a hair-care product suitable for hair application and/or at least temporary retention thereupon.

According to a further aspect of some embodiments of the disclosure, there is provided a method of protecting the surface of an inanimate object against ultraviolet radiation, the method comprising applying to the surface of the object an efficacious amount of a UV protective composition comprising doped or undoped zinc titanate crystals. In some such embodiments, the UV-protective composition can be in the form of a coating product suitable for application to inanimate surfaces and/or at least temporary retention thereupon.

According to a further aspect of some embodiments of the disclosure, there is provided the use of doped or undoped zinc titanate crystals in the manufacture of a composition for protection of the skin of a subject against ultraviolet radiation.

According to a further aspect of some embodiments of the disclosure, there is provided the use of doped or undoped zinc titanate crystals in the manufacture of a composition for protection of the hair of a subject against ultraviolet radiation.

According to a further aspect of some embodiments of the disclosure, there is provided the use of doped or undoped zinc titanate crystals in the manufacture of a composition for protection of surfaces of an object against ultraviolet radiation.

According to a further aspect of some embodiments of the disclosure, there is provided a method of manufacturing a UV protective composition, comprising combining doped or undoped zinc titanate crystals, as an ultraviolet-absorbing agent, with other ingredients in proportions and in a manner suitable to make a UV-protective composition as described herein.

In some embodiments of the composition, use or method disclosed herein, the zinc titanate crystals are present in the composition at a concentration of from about 0.001% (w/w) to about 40% (w/w), such as about 0.1, 1, 2, 3, 4, 5, 10, 15, 20, 25, 30 or 35% (w/w), from about 0.01% (w/w) to about 30% (w/w), from about 0.1% (w/w) to about 20% (w/w) or from about 0.1% (w/w) to about 15% (w/w) of the final composition.

In some embodiments, the zinc titanate crystals constitute at least 0.01 wt.%, at least 0.1 wt.%, at least 0.5 wt.%, at least 1 wt.%, at least 2 wt.%, at least 3 wt.%, at least 4 wt.%, at least 5 wt.%, at least 10 wt.%, at least 15 wt.%, at least 20 wt.%, at least 25 wt.%, at least 30 wt.%, or at least 35 wt.% of the composition. In some embodiments, the zinc titanate crystals constitute at most 40 wt.%, at most 35 wt.%, at most 30 wt.%, at most 25 wt.%, at most 20 wt.%, at most 15 wt.%, at most 10 wt.%, at most 5 wt.%, at most 4 wt.%, at most 3 wt.%, at most 2 wt.%, at most 1 wt.%, at most 0.5 wt.%, or at most 0.1 wt.% of the composition.

In some embodiments of the composition, use or method disclosed herein, the doped or undoped zinc titanate crystals are present in the composition as nanoparticles having at least one dimension of up to about 200 nm, such as 10, 20, 30, 40, 50, 60, 70, 80, 90, 100, 110, 120, 130, 140, 150, 160, 170, 180 or 190 nm. In some embodiments, the nanoparticles have at least one dimension in the range of from about 10 nm to about 200 nm, from about 20 nm to about 150 nm, from about 20 to about 100 nm, from about 10 nm to about 80 nm, from about 10 to about 70 nm, from about 20 to about 70 nm, or from about 20 to about 60 nm, In some particular embodiments, the nanoparticles have at least one dimension of about 30 nm.

In some embodiments, the afore-mentioned dimensions or ranges of dimensions apply to at least 95%, or at least 97.5% or at least 99% of the population of the nanoparticles.

In some embodiments, the aforesaid smallest dimension of doped or undoped zinc titanate crystals is estimated based on the hydrodynamic diameter of the particles as measured by DLS techniques. In some embodiments, the population distribution of the particles is expressed in terms of the cumulative particle size distribution, according to the number of particles in a sample. In some embodiments, the population distribution of the particles is expressed in terms of the cumulative particle size distribution of a sample volume of particles.

In some embodiments of the composition, use or method disclosed herein, the composition is generally devoid and/or generally free of an organic ultraviolet-absorbing agent.

In some embodiments of the composition, use or method disclosed herein, the composition is generally free of an organic ultraviolet-absorbing agent, that is to say the composition contains less than 5 wt.% organic UV-absorbing agents. In some embodiments the composition contains less than 4 wt.%, less than 3 wt.%, less than 2 wt.% or less than 1 wt.% organic UV-absorbing agents. In some embodiments the composition is largely free of organic ultraviolet-absorbing agents, *i.e.* the composition contains less than 0.5 wt.% organic UV-absorbing agents. In some embodiments the composition is mostly free of organic UV-absorbing agents, *i.e.* the composition contains less than 0.1 wt.% organic UV-absorbing agents. In some embodiments the composition is principally free of organic ultraviolet-absorbing agents, *i.e.* the composition contains less than 0.05 wt.% organic UV-absorbing agents. In some embodiments the composition is fundamentally free of organic UV-absorbing agents, *i.e.* the composition contains less than 0.01 wt.% organic UV absorbing agents. In some embodiments of the composition, use or method disclosed herein, the composition is generally devoid of organic ultraviolet-absorbing agents, considerably devoid of organic ultraviolet-absorbing agents, significantly devoid of organic ultraviolet-absorbing agents, substantially devoid of organic ultraviolet-absorbing agents, essentially devoid of organic ultraviolet-absorbing agents, substantively devoid of organic ultraviolet-absorbing agents or devoid of organic ultraviolet-absorbing agents.

In some embodiments of the composition, use or method disclosed herein, the composition is generally devoid and/or generally free of an additional inorganic ultraviolet-absorbing agent.

In some embodiments of the composition, use or method disclosed herein, the composition is generally free of an additional inorganic ultraviolet-absorbing agent, that is to say the composition contains less than 5 wt.% additional inorganic UV-absorbing agents. In some embodiments the composition contains less than 4 wt.%, less than 3 wt.%, less than 2 wt.% or less than 1 wt.% additional inorganic UV-absorbing agents. In some embodiments the composition is largely free of additional inorganic ultraviolet-absorbing agents, *i.e.* the composition contains less than 0.5 wt.% additional inorganic UV-absorbing agents. In some embodiments the composition is mostly free of additional inorganic UV-absorbing agents, *i.e.* the composition contains less than 0.1 wt.% additional UV-absorbing agents. In some embodiments the composition is principally free of additional inorganic ultraviolet-absorbing agents, *i.e.* the composition contains less than 0.05 wt.% additional UV-absorbing agents. In some embodiments the composition is fundamentally free of additional inorganic UV-absorbing agents, *i.e.* the composition contains less than 0.01 wt.% additional UV absorbing agents.

In some embodiments of the composition, use or method disclosed herein, the composition is generally devoid of additional ultraviolet-absorbing agents, considerably devoid of additional ultraviolet-absorbing agents, significantly devoid of additional ultraviolet-absorbing agents, substantially devoid of additional ultraviolet-absorbing agents, essentially additional of organic ultraviolet-absorbing agents, substantively devoid of additional ultraviolet-absorbing agents or devoid of additional ultraviolet-absorbing agents.

In some embodiments of the composition, use or method disclosed herein, the doped or undoped zinc titanate crystals are the sole ultraviolet-absorbing agent.

In some embodiments of the composition, use or method disclosed herein, the composition further comprises silver metal particles.

In some embodiments, the silver metal particles are present in the composition as nanoparticles. In some embodiments, the silver nanoparticles have at least one dimension of up to about 50 nm. In some embodiments, the silver nanoparticles have at least one dimension of up to about 40 nm. In some embodiments, the silver nanoparticles have at least one dimension of up to about 30 nm. In some embodiments, the silver nanoparticles have at least one dimension in the range of from about 10 nm to up to about 50 nm.

In some embodiments, the afore-mentioned dimensions or ranges of dimensions apply to at least 90%, or at least 95%, or at least 97.5% or at least 99% of the population of the silver nanoparticles.

In some embodiments, the aforesaid at least one dimension of the silver nanoparticles is estimated based on the hydrodynamic diameter of the particles as measured by DLS techniques. In some embodiments, the population distribution of the particles is expressed in terms of the cumulative particle size distribution according to the number of particles in a sample. In some embodiments, the population distribution of the particles is expressed in terms of the cumulative particle size distribution of a sample volume of particles.

In some embodiments, the silver nanoparticles are present in the composition at a concentration in the range of from about 0.01% to about 10% (w/w) of the total composition, such as about 0.1, 1, 2, 3, 4, 5, 6, 7, 8, or 9% (w/w) of the total composition. In some embodiments, the silver nanoparticles are present in the composition at a concentration in the range of from about 0.01% to about 5% (w/w), from about 0.05% to about 5% (w/w), or from about 0.1% to about 2% (w/w) of the total composition. In some preferred embodiments, the silver nanoparticles are present in the composition at a concentration of about 1% (w/w) or about 2% (w/w) of the total composition.

In some embodiments, the silver particles constitute at least 0.01 wt.%, at least 0.1 wt.%, at least 0.5 wt.%, at least 1 wt.%, at least 2 wt.%, at least 3 wt.%, at least 4 wt.%, at least 5 wt.% or at least 10 wt.% of the composition. In some embodiments, the silver particles constitute at most 10 wt.%, at most 5 wt.%, at most 4 wt.%, at most 3 wt.%, at most 2 wt.%, at most 1 wt.%, at most 0.5 wt.%, or at most 0.1 wt.% of the composition.

In some embodiments of the composition, use or method disclosed herein, the UV protective composition is a composition for human or animal use, formulated as a topical composition. The topical composition may optionally be provided in a form selected from the group consisting of a cream, an emulsion, a gel, a lotion, a mousse, a paste and a spray. If desired, the topical composition can also be formulated into make-up cosmetics, for example, foundation, blusher, *etc.*

In some embodiments, the topical composition further comprises a dermatologically or cosmetically or pharmaceutically acceptable carrier.

In some embodiments, the topical composition further comprises one or more dermatologically or cosmetically or pharmaceutically acceptable additives or excipients, such as colorants, preservatives, fragrances, humectants, emollients, emulsifiers, waterproofing agents, surfactants, dispersants, thickeners, viscosity modifiers, anti-foaming agents, conditioning agents, antioxidants and the like. Such additives or excipients and the concentrations at which each can effectively accomplish its respective functions, are known to persons skilled in the pertinent art and need not be further detailed.

In some embodiments, the topical composition is a sunscreen composition.

In some embodiments, the UV protective composition is in the form of a coating that can be applied to the surface of an inanimate object. The coating composition may be provided in a form selected from the group consisting of liquid coat, an emulsion, a cream, a gel, a paste, a film, a powder and a spray.

In another aspect of the present disclosure, there is provided a method for the preparation of the compositions disclosed herein.

According to a further aspect of some embodiments of the disclosure, there is provided a UV protective composition as disclosed herein, for use in protecting a subject, such as a human subject or a non-human animal, against an effect of ultraviolet radiation, in some embodiments providing broad-spectrum protection against both ultraviolet A and ultraviolet B radiation.

In some embodiments, the composition is for use in protecting the skin of a subject, against an effect of ultraviolet radiation, in some embodiments providing broad-spectrum protection against both ultraviolet A and ultraviolet B radiation.

In some embodiments, the composition is for use in protecting the hair of a subject, such as a human subject, against an effect of ultraviolet radiation, in some embodiments against effects of both ultraviolet A and ultraviolet B radiation.

The skin may be the skin of the face, of the arms, of the legs, of the neck of the torso, or of any other area of the body that can be exposed to UV radiation.

In some embodiments, the sunscreen composition as disclosed herein is applied to the skin of the subject prior to or during exposure to UV radiation. In some embodiments, the composition is reapplied intermittently, for example every 10 hours, every 9 hours, every 8 hours, every 7 hours, every 6 hours, every 5 hours, every 4 hours, every 3 hours, every 2 hours or every hour, or any intermediate value, during exposure to UV radiation.

In some embodiments, the UV-protective composition is for protecting the hair of a subject against ultraviolet radiation and is provided in a form selected from the group consisting of a cream, an emulsion, a gel, a lotion, a mousse, a paste and a spray. In some embodiments, the composition is provided in the form of a shampoo, a conditioner or a hair mask.

In some embodiments, the composition is formulated to be applied to the hair, or is applied to the hair, for a fixed period of time (such as up to 1 minute, up to 2 minutes, up to 3 minutes, up to 4 minutes or up to 5 minutes, up to 10 minutes, up to 15 minutes, up to 20 minutes, up to 25 minutes or up to 30 minutes) prior to rinsing. In some embodiments, the conditioner or hair mask is formulated for application to the hair, or is applied to the hair without rinsing, such that the conditioner or hair mask remains on the hair.

According to a further aspect of some embodiments of the disclosure, there is provided a UV protective composition as disclosed herein, for use in protecting an inanimate object, against an effect of ultraviolet radiation, in some embodiments providing broad-spectrum protection against both ultraviolet A and ultraviolet B radiation.

According to a further aspect of some embodiments of the disclosure, there is provided a method of protecting the skin or the hair of a subject against an effect of ultraviolet radiation, the method comprising applying to the skin and/or the hair of the subject a sunscreen composition comprising a matrix comprising a polymer and an oil; and particles of doped or undoped zinc titanate crystals, dispersed in the matrix.

According to a further aspect of some embodiments of the disclosure, there is provided the use of a matrix comprising a polymer and an oil; and particles of a UV protective-agent comprising doped or undoped zinc titanate crystals, dispersed in the matrix, in the manufacture of a composition for protection of the skin and/or the hair of a subject against an effect of ultraviolet radiation.

According to a further aspect of some embodiments of the disclosure, there is provided the use of a matrix comprising a polymer and an oil; and particles of a UV protective-agent comprising doped or undoped zinc titanate crystals, dispersed in the matrix, in the manufacture of a composition for protection of exterior surfaces of an inanimate object against an effect of ultraviolet radiation. The exterior surface may comprise the surface of any porous or non-porous material, including, but not limited to glass, fabrics, leathers, woods, cardboards, metals, plastics, rubbers, ceramics and other structural materials.

The composition for the protection of inanimate objects against UV radiation, can be formulated in any form suitable for application to the surface of the inanimate object on which it is to be used.

### EXAMPLES

### Materials and Methods

### Materials

The following materials were purchased from Sigma Aldrich, USA:

| | |
|---|---|
| ZnO (99.9% pure) | CAS 1314-13-2 |
| TiO₂ (99% pure) | CAS 13463-67-7 |
| Fe₂O₃ (99% pure) | CAS 1309-37-1 |
| Poly Acrylic Acid Sodium base (PAA) | CAS 9003-04-7 |

The milling media, namely Zirconia beads having an average diameter of 2mm, were purchased from Pingxiang Lier Ceramic Co., China.

### Equipment

High Resolution Scanning Electron Microscope HSEM/TEM Magellan XHR 400L FE-SEM by Nanolab Technologies, Albany, New York, USA.
High Resolution X-ray diffractometer XRD Rigaku SmartLab® with Cu radiation generated at 40 kV and 30 mA (CuKa= 1.542 A) as the X-ray source.
Particle Size Analyser (Light Scattering) Zen 3600 Zetasizer by Malvern Instruments, Malvern, UK
Oven, Vulcan-Hart 3-1750 multi-stage programmable box furnace.
Temperature controllable circulating water bath, BL-30L 9 liter 1/3HP by MRC, Hampstead, London, UK
Grinding Mill Model HD-01 Attritor by Union Process®, Inc., Akron, Ohio, USA.
Analytical Balance XSE by Mettler-Toledo International Inc., Columbus, Ohio, USA.
Mortar Grinder Pulverisette 2 by Fritsch GmbH, Idar-Oberstein, Germany.
Double Planetary Mixer by Charles Ross & Son Company, Hauppauge, New York, USA.

### Example 1: Preparation of zinc titanate crystals

Doped and undoped zinc titanate crystals having the general formula Zn₂Ti₍₁₋ₓ₎FeₓO₄ wherein x is from 0 to 0.1, were prepared by a solid solution method. The Fe-doped crystals included two molar ratios Ti:Fe 0.975:0.025 and 0.95:0.05 (*i.e.* wherein x = 0.025 or 0.05, respectively).

In this process, the constituent metal oxides were mixed together in powder form so as to obtain the desired stoichiometric amount. ZnO, having a MW of 81.4084g/mol and TiO₂ having a MW of 79.87g/mol were mixed in desired ratio so that the combined ZnTiO₄ powder amounted to about 200 grams. When desired, Fe₂O₃ having a MW of 159.69g/mol, was added while the amount of titanium dioxide was reduced, the amount of ferric oxide selected to provide the required doping ratio. The powder due to be iron doped amounted likewise to about 200 grams.

All materials were weighed using an analytical scale (Mettler Toledo, USA).

The powders of the constituent reagents were then mixed together for about 10 minutes at 70 rpm at ambient temperature in a Pulverisette 2 mortar grinder (Fritsch, Germany), so as to obtain homogeneously mixed presintered powders (to be doped or undoped, as appropriate). The mixed powders were transferred to a 500ml alumina crucible and sintered or calcined by heating in a ceramic oven at a rate of 40°C per minute until the temperature reached 1000°C, and maintained at this temperature for 24 hours, allowing for the formation of the desired doped or undoped zinc titanate crystals. It is believed that under such conditions, the iron atoms can substitute the titanium atoms in the orthorhombic structure of the zinc titanate crystals to provide doping without breaking the crystallographic symmetry.

After 24 hours at 1000°C, the samples were allowed to cool down to ambient temperature (*circa* 23°C), at which time they were again ground to homogeneous powder for about 10 minutes at 70 rpm by the Pulverisette 2 mortar grinder.

Powders of doped or undoped zinc titanate crystals prepared as above-described were either used or analyzed "as is" in coarse form, or further size-reduced and used and analyzed in the form of nanoparticles, as described in following examples. It is to be understood that the coarse material was manually ground with a mortar and pestle to disassociate any gross agglomerate that may be present in the resulting powders, so as to eliminate coarse lumps of particles. In bulk size, the zinc titanate compounds displayed a white shade if undoped and a pale reddish tint if doped, the color intensity depending on the degree of iron doping.

### Example 2: Absorbance Determination in Powder

Absorbance correlation of coarse powders over the wavelength range of 200--800 nm was calculated using a Cary 300 UV-Vis spectrophotometer with an integrated sphere detector (Agilent Technologies, Santa Clara, CA, USA).

Briefly, the absorbance of the samples was qualitatively estimated by subtracting the amount of light reflected from the powder sample, gathered by the integrated sphere detector of the spectrophotometer, from the amount of light reflected from a white surface (which reflects all incident light). Since the extent of penetration of the light into the samples and the extent of scattering of the sample is unknown, this measurement provides an absorbance profile of the sample rather than a true quantitative measurement.

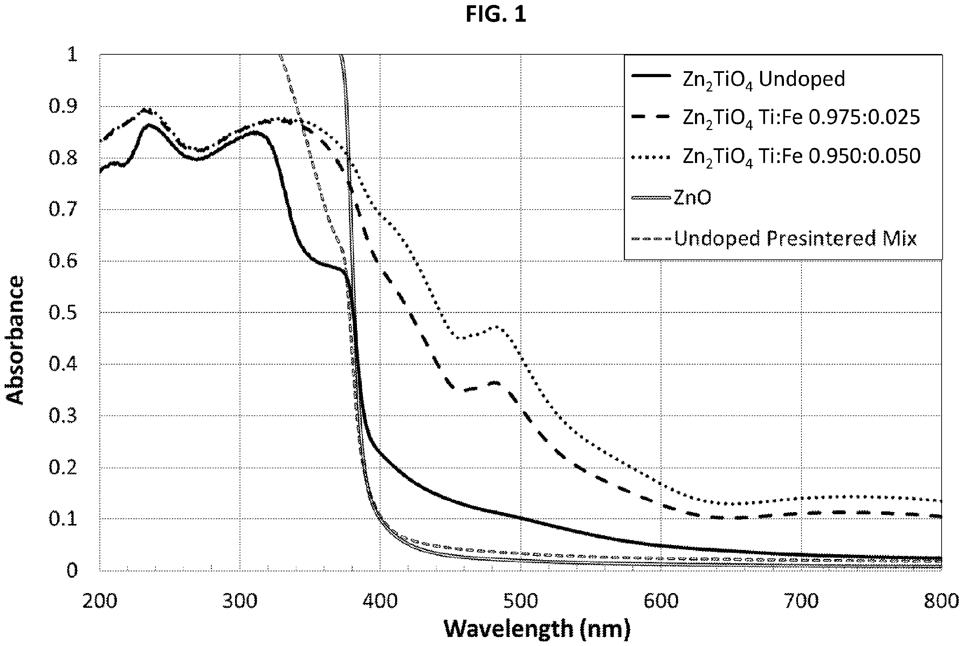

Results, showing correlation to absorbance as a function of wavelength, determined by diffuse reflection measurement gathered by the integrated sphere method, are presented in Figure 1.

**Figure 1** shows the absorbance of doped (Ti:Fe 0.975:0.025 or 0.95:0.05) or undoped (x=0) zinc titanate crystals, as obtained following the sintering method of Example 1 as compared to undoped zinc oxide or to an undoped presintered mixture of zinc oxide and titanium dioxide in appropriate stoichiometric amounts.

As seen in Figure 1, undoped zinc oxide exhibits a very sharp decrease in UV absorbance in the range of from about 380 nm to about 400 nm. The presintered mixture of zinc oxide and titanium dioxide corresponding to the undoped zinc titanate displayed an absorbance pattern similar to zinc oxide alone with a sharp decrease at 380 nm. Undoped zinc titanate, differing from its presintered version, has a relatively constant UV absorbance from 200 nm to about 310 nm, with a gradual decrease in the range of from about 310 nm to about 380 nm, followed by a sharper decrease at about 380 nm, but providing higher absorbance levels than undoped zinc oxide (or its presintered mix) in the range of from about 380 nm to about 400 nm. Crystals of doped zinc titanate (Ti:Fe 0.975:0.025 or 0.95:0.05) exhibited significantly higher UV absorbance than either undoped zinc oxide or undoped zinc titanate crystals in the 380 nm to 400 nm range, with absorbance of Ti:Fe 0.95:0.05 doped zinc titanate crystals being higher than that of the Ti:Fe 0.975:0.025 doped equivalent.

### Example 3: Crystal Structure Determination

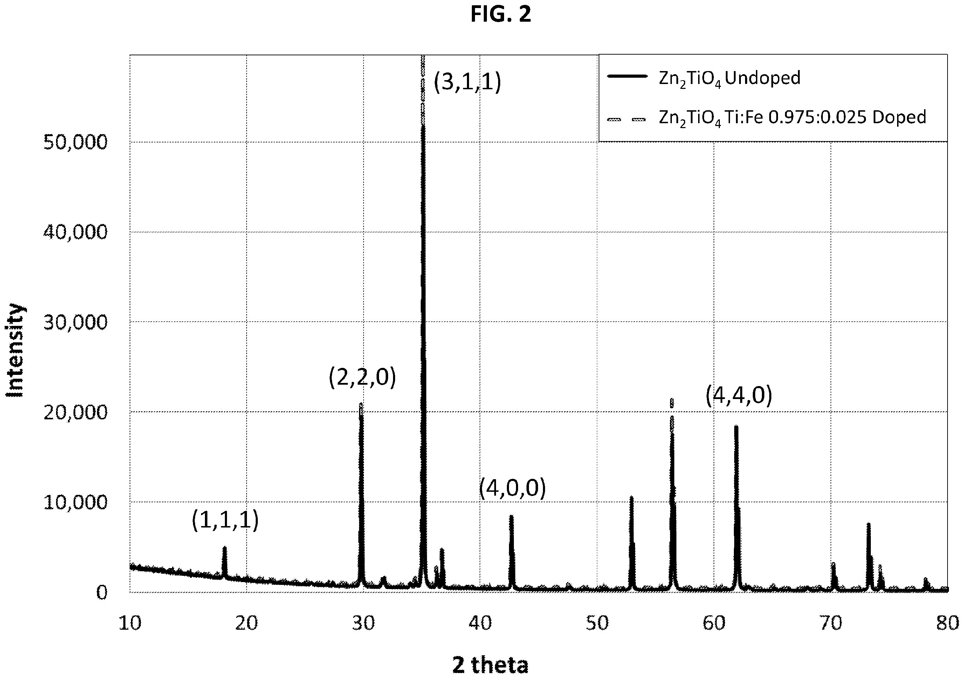

The crystal structure of undoped or doped (Ti:Fe 0.975:0.025) zinc titanate, as above-prepared, was determined by powder XRD using Rigaku TTRAX-III X-ray diffractometer. The X-ray source (Cu anode) was operated at a voltage of 40 kV and a current of 30 mA on packed powder samples. Data were collected in continuous detector scan mode at a step size of 0.02°/step. Diffractograms were collected over the 2Θ range of 10° to 80°. The results are shown in **Figure 2****,** wherein the pattern of undoped zinc titanate crystals is displayed as a continuous line, whereas that of the doped equivalent is shown as a dotted line. For both materials, a predominant peak is seen around 2Θ of about 35° and doping did not significantly affect the crystalline peaks characteristic of the zinc titanate crystals, the main ones being indicated on the figure.

### Example 4: Preparation of nanoparticles

Nanoparticles of doped (Ti:Fe 0.975:0.025 or 0.95:0.05) or undoped zinc titanate crystals were prepared from the ground sintered samples obtained in Example 1. Nanoparticles of zinc oxide were prepared for comparison from its stock powder. Generally, all such samples or stock powders contained particles having a size greater than about 5 micrometer (µm) and may be referred hereinafter as the coarse materials. The coarse powders were milled in an Attritor grinding mill (HD-01 by Union Process®) using a batch size of 200g with solid loading 10% (20g) as follows.

All materials were weighed using an analytical scale (XSE by Mettler Toledo). 20 g of PAA dispersant was weighed and dispersed in about 100 ml of deionized water. 20 g of coarse powder was weighed and introduced into the dispersant-containing liquid to provide a dispersant to inorganic material ratio of 1:1 yielding a slurry of the inorganic material. Water was added to complete batch size to 200g, the solids constituting about 10wt.% of the sample.

The aqueous slurry of inorganic material was then placed in a zirconia pot with 2300g of 2mm diameter zirconia grinding beads. The pot was placed in the grinding mill, and the grinding mill activated at 700 RPM for about 75 hours at 25°C.

The hydrodynamic diameter of the nanoparticles obtained by this method was determined by Dynamic Light Scattering, using a Zen 3600 Zetasizer from Malvern Instruments Ltd. (Malvern, UK). A sample of the milled nanoparticles was further diluted in deionized water to form a suspension having a solid concentration of about 0.5 wt.%.

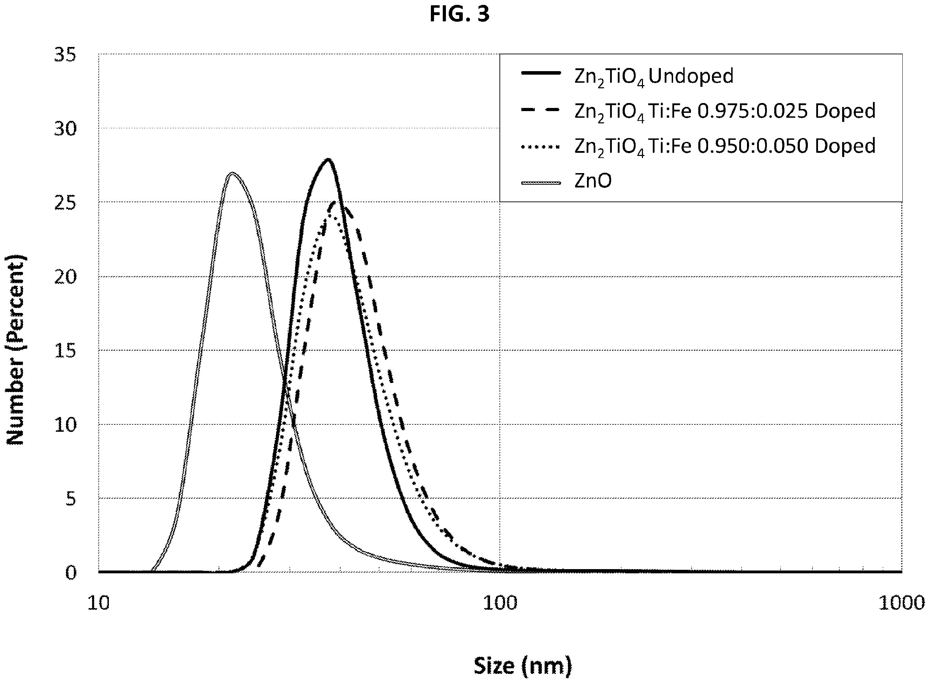

Representative results, showing the percentage of number of doped (Ti:Fe 0.975:0.025 and 0.95:0.05) and undoped zinc titanate crystal particles, as well as zinc oxide as reference, having hydrodynamic diameters in the range of 10-1000 nm are presented in Figure 3.

**Figure 3** shows that the majority of doped and undoped zinc titanate crystal particles had hydrodynamic diameters in the size range of from about 20 nm and up to about 100 nm. The predominant peaks of doped (Ti:Fe 0.975:0.025 and 0.95:0.05) and undoped zinc titanate crystals were each at around 40 nm. Results of the particle size distribution of the nanoparticles prepared as herein described, namely the maximum hydrodynamic diameter of a percentage of the population, are provided in the Table 1 below, in terms of percent of number of particles. Information on zinc oxide is provided for reference.

**Table 1**

| | **Max. Hydrodynamic Diameter (nm)** | | | | | |
|---|---|---|---|---|---|---|
| **Material** | 10% | 50.0% | 90.0% | 95.0% | 97.5% | 99.0% |
| **Zinc oxide** | 20.2 | 26.4 | 36.2 | 39.5 | 47.7 | 62.2 |
| **Zn₂TiFeO₄ (Fe:Ti 0.025:0.975)** | 29.8 | 40.4 | 60.5 | 70.7 | 83.5 | 110 |
| **Material** | 10% | 50.0% | 90.0% | 95.0% | 97.5% | 99.0% |
| **Zn₂TiFeO₄ (Fe:Ti 0.05:0.95)** | 31.9 | 42.4 | 62.2 | 71.3 | 82.4 | 104 |
| **Zn₂TiO₄ ref** | 29.2 | 38 | 53.7 | 60.7 | 70.2 | 103 |

As can be seen from the above table, at least 97.5% of the nanoparticles of doped or undoped zinc titanate crystals as prepared and size-reduced according to the present teachings have a dimension of at most 100 nm.

### Example 5: Absorbance of suspended crystal nanoparticles

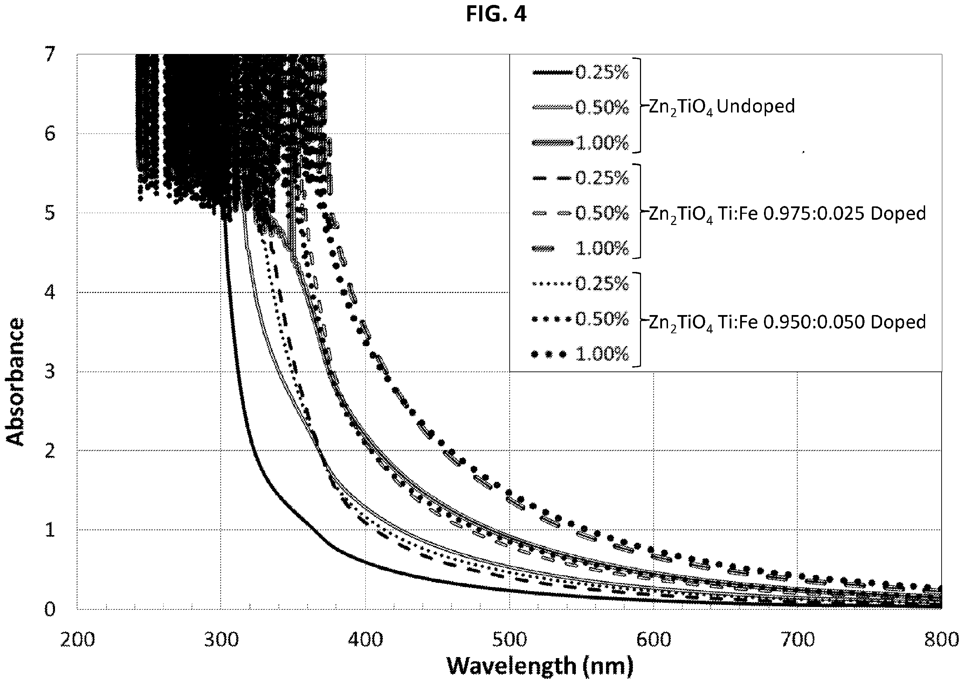

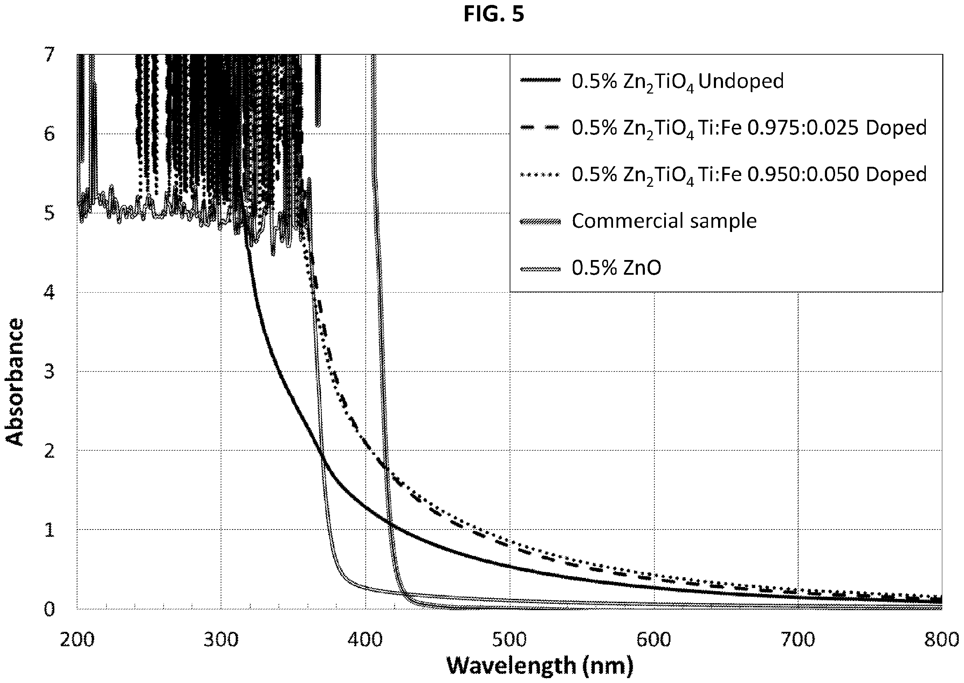

Absorbance of the nanoparticles of doped and undoped zinc titanate crystals prepared according to Example 4 was measured over the wavelength range of 200-800 nm using a Cary 300 UV-Vis spectrophotometer with quartz cuvette (10mm light pathway). The samples were diluted in the vehicle in which the inorganic materials were milled (namely with deionized water containing 10wt.% PAA) to provide any desired predetermined solid concentration (e.g., 0.25 wt.%, 0.5 wt.%, and 1.0 wt.%,). Results are presented in Figures 4 and 5. For convenience, it should be recalled that an absorbance value of 1 indicates a UV blocking of at least about 90%, whereas an absorbance value of 2 indicates blocking of up to 99% of the radiation.

In **Figure 4****,** the absorbance in the 200-800 nm wavelength range is shown for nanoparticles of undoped zinc titanate crystals nanoparticles and for 0.975:0.025 Ti:Fe and 0.95:0.05 Ti:Fe doped zinc titanate nanoparticles at three concentrations of 0.25 wt.%, 0.5 wt.% and 1 wt.%.

As can be seen in the figure, doped and undoped zinc titanate crystals displayed significant absorbance up to at least 360 nm at all concentrations tested, with all materials except for 0.25 wt.% undoped zinc titanate crystals displaying substantial absorbance at 400 nm. Absorbance across the tested range was shown to increase with increasing zinc titanate concentration and degree of doping at the concentrations and Fe:Ti ratios tested.

**Figure 5** shows absorbance of undoped zinc titanate crystals, doped (Ti:Fe 0.975:0.025 or 0.95:0.05) zinc titanate crystals, and zinc oxide as reference, each at a concentration of 0.5 wt.%. As shown in the figure, zinc oxide displayed an insignificant level of absorbance at wavelengths of higher than about 380 nm, displaying at 400 nm an absorbance of about 0.26. For comparison, undoped zinc titanate crystals displayed an absorbance of about 1.3 at 400 nm, while the doped variants each displayed absorbance of at least 2.1 at 400 nm. A commercial sunscreen composition (Skingard® sunscreen composition by Careline® (Pharmagis, Israel)) based on organic UV blockers was included for convenient comparison.

### Example 6: Scanning electron microscope studies

The doped and undoped zinc titanate crystal nanoparticles were also studied by High Resolution Scanning Electron Microscopy (HR-SEM) using Magellan™ 400 HSEM/TEM by Nanolab Technologies.

**Figure 6A** shows an image for undoped zinc titanate crystal nanoparticles, wherein **Figure 6B** shows an image for Fe-doped zinc titanate crystal nanoparticles (Ti:Fe 0.95:0.05).

As shown in the figures, doped and undoped zinc titanate crystal particles having spheroid shape with diameters of less than about 100 nm, mainly less than about 70 nm, were obtained. Larger clusters are deemed non-representative, resulting from agglomeration of individual particles upon preparation of the sample for HR-SEM analysis, the drying out of the liquid carrier being known to cause such artificial outcome. The good correlation between the diameters of the particles when measured in suspension and in dried form confirm the suitability of the above-described method to prepare nanoparticles having at least one dimension (e.g. a diameter) of up to about 100 nm.

### Example 7: Determination of critical wavelength

Based on the absorbance spectra determined according to previous Examples, critical wavelength was calculated for undoped zinc titanate crystals and for two Fe-doped variants (Ti:Fe 0.975:0.025 and 0.95:0.05), all measured at nanoparticle concentration of 0.25wt.%, 0.5wt.% and 1wt.%. A suspension of nanoparticles of Zinc Oxide at 0.5wt.% served as control.

Briefly, in order to quantify the breadth of UV protection, the absorbance of the sunscreen composition was integrated from 290 nm to 400 nm the sum reached defining 100% of the total absorbance of the sunscreen in the UV region. The wavelength at which the summed absorbance reaches 90% absorbance was determined as the 'critical wavelength' which provided a measure of the breadth of sunscreen protection.

The critical wavelength λ_{c} was defined according to the following equation: ${\int }_{290}^{{λ}_{c}} Ig \left[1/T\left(λ\right)\right] d λ = 0.9 ⋅ {\int }_{290}^{400} Ig \left[1/T\left(λ\right)\right] d λ$ wherein:
λ_{c} is the critical wavelength;
*T*(λ) is the mean transmittance for each wavelength; and
Dλ is the wavelength interval between measurements.

Critical wavelengths as calculated are presented in Table 2 below.

**Table 2**

| | **Critical Wavelength (nm)** | | |
|---|---|---|---|
| **Inorganic Material** | 0.25wt.% | 0.5wt.% | 1wt.% |
| **Zinc titanate undoped** | 372 | 377 | 381 |
| **Fe-doped zinc titanate Ti:Fe 0.975:0.025** | 373 | 379 | 383 |
| **Fe-doped zinc titanate Ti:Fe 0.95:0.05** | 373 | 380 | 385 |
| **ZnO Control** | - | 362 | - |

As can be seen from the above table, according to the Critical Wavelength Method, undoped and Fe-doped zinc titanate crystal nanoparticles can be classified as providing broad spectrum protection (*i.e*. having a critical wavelength of 370 nm or more) at concentrations of as low as 0.25wt.%. Such results are superior to those achieved by the control suspension consisting of ZnO nanoparticles having similar particle size distribution which even when tested at the concentration of 0.5wt.% displayed a narrower spectrum protection, its critical wavelength being of only 362 nm.

### Example 8: Preparation of composition comprising polymer matrix and zinc titanate

The nanoparticles of doped or undoped zinc titanate crystals prepared according to the present teachings and above-examples can be further processed so as to be embedded or immobilized within a polymer matrix. Suitable methods and polymers are described by the present Applicant in PCT Publication No. WO 2017/013633, incorporated herein by reference in its entirety as if fully set forth herein. In particular, Example 2 of the reference provides for the preparation of a polymer matrix, whereas Example 3 teaches how to blend such matrix with nanoparticles, and how to further process such mixture so as to obtain polymer embedded particles. Anon-limiting example of a suitable polymer matrix comprises Nucrel® (methylene-methacrylic acid copolymer) of DuPont, USA, dispersed in Isopar® (paraffinic oil) of ExxonMobil Chemical Company, USA.

### Example 9: Preparation of composition comprising zinc titanate in wood lacquer

Doped and undoped zinc titanate crystal nanoparticles are diluted in a clear wood lacquer (Tambour Clear Glossy Lacquer for Wood No. 8, Cat. No. 149-001) to a particle concentration of 1% by weight of the total lacquer composition. The resulting mixtures are sonicated for 30 seconds using a Misonix Sonicator tip (Misonix, Inc.) at amplitude 100, 15 W. The sonicated lacquer dispersions are applied upon a microscopic glass slide at an initial thickness of about 100 µm (using 100 µm thick spacers and a leveling rod). The lacquer coated slides are left to dry for at least 12 hours at ambient temperature (*circa* 23°C) resulting in a dried layer of sample of about 5 µm. The lacquer devoid of added nanoparticles serves as control. Absorbance of the dried layers of lacquer over the wavelength range of 200--800 nm is assessed using a Cary 300 UV-Vis spectrophotometer.

Although the disclosure has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

Citation or identification of any reference in this application shall not be construed as an admission that such reference is available as prior art to the disclosure.

## Claims

1. A UV-protective composition comprising one or more zinc titanate crystals each independently having the chemical formula Zn₂Ti₍₁₋ₓ₎FeₓO₄, wherein x is between 0.005 and 0.1, wherein the zinc titanate crystals are in the form of nanoparticles consisting of one or more said crystals, at least 50% of the total number of said nanoparticles having at least one dimension of up to 200 nm.

2. The UV-protective composition according to claim 1, wherein x is between 0.025 and 0.05.

3. The UV-protective composition according to any one of claim 1 or claim 2, wherein at least 90% of the total number of said nanoparticles have at least one dimension of up to about 200 nm.

4. The UV-protective composition according to any one of claim 1 to claim 3, wherein the one or more zinc titanate crystals are at a concentration in the range of from about 0.001% to about 40% (w/w) of the composition.

5. The UV-protective composition according to any one of claim 1 to claim 4, wherein said nanoparticles of said one or more zinc titanate crystals are dispersed with a dispersant in a polymer matrix comprising an oil-swellable thermoplastic polymer in an oil-based carrier.

6. The composition according to claim 5, wherein said polymer matrix is in the form of polymer matrix flakes, wherein each flake of said polymer matrix flakes has a flake length (Lf), a flake width (Wf), and a flake thickness (Tf), said polymer matrix flakes having a dimensionless flake aspect ratio (Rf) defined by: Rf = (Lf•Wf)/(Tf)²; wherein, with respect to a representative group of at least ten polymer matrix flakes, an average Rf is at least 5; and wherein the nanoparticles within said representative group have an cumulative particle size (D50) of at most 100 nm.

7. The UV-protective composition according to any one of claim 5 or claim 6, wherein the dispersant adapted to disperse the nanoparticles of the zinc titanate crystals within said polymer matrix has a hydrophilic-lipophilic balance (HLB) value of at most 9.

8. The UV-protective composition according to any one of claim 5 to claim 7, wherein the thermoplastic polymer in the polymer matrix comprises at least one of an ethylene-acrylic (EAA) polymer, an ethylene-methacrylic (EMMA) polymer, an ethyl vinyl acetate (EVA) polymer, or combinations thereof.

9. The UV-protective composition according to any one of claim 1 to claim 8, formulated as one or more of the following: (a) a skin-care composition for application to human or non-human animal skin; (b) a hair-care composition for application to human or non-human animal hair; or (c) a coating composition for application to an inanimate surface.

10. The UV-protective composition according to any one of claim 1 or claim 2, wherein a molar ratio of Ti to Fe is 19 to 1.

11. The UV-protective composition according to any one of claim 1 to claim 10, wherein the absorption spectrum of the composition has a critical wavelength of at least 370 nm.

12. The UV-protective composition of any one of claim 1 to claim 11, wherein the area under the curve (AUC) formed by the UV-absorption of the zinc titanate crystals as a function of wavelength in the range of 280 nm to 400 nm (AUC₂₈₀₋₄₀₀) is at least 75% of the AUC formed by the same zinc titanate crystals at the same concentration in the range of 280 nm to 700 nm (AUC₂₈₀₋₇₀₀).

13. Use of the UV-protective composition according to any one of claim 1 to claim 12, for protecting the hair of a subject or for protecting an inanimate object against an effect of ultraviolet radiation, in particular protecting against ultraviolet A radiation and ultraviolet B radiation.

14. The UV-protective composition according to any one of claim 1 to claim 12, for use in protecting the skin of a subject against an effect of ultraviolet radiation.

15. The UV-protective composition for use according to claim 14, wherein protecting against ultraviolet radiation comprises protecting against ultraviolet A radiation and ultraviolet B radiation.

## Patentansprüche

1. UV-Schutzzusammensetzung, umfassend einen oder mehrere Zinktitanatkristalle, die jeweils unabhängig die chemische Formel Zn₂Ti₍₁₋ₓ₎FeₓO₄ aufweisen, wobei x zwischen 0,005 und 0,1 liegt, wobei die Zinktitanatkristalle in der Form von Nanopartikeln sind, die aus einem oder mehreren der Kristalle bestehen, wobei zumindest 50 % der Gesamtzahl der Nanopartikel zumindest eine Abmessung von bis zu 200 nm aufweisen.

2. UV-Schutzzusammensetzung nach Anspruch 1, wobei x zwischen 0,025 und 0,05 liegt.

3. UV-Schutzzusammensetzung nach einem von Anspruch 1 oder Anspruch 2, wobei zumindest 90 % der Gesamtzahl der Nanopartikel zumindest eine Abmessung von bis zu etwa 200 nm aufweisen.

4. UV-Schutzzusammensetzung nach einem von Anspruch 1 bis Anspruch 3, wobei der eine oder die mehreren Zinktitanatkristalle in einer Konzentration im Bereich von etwa 0,001 % bis etwa 40 % (w/w) der Zusammensetzung vorliegen.

5. UV-Schutzzusammensetzung nach einem von Anspruch 1 bis Anspruch 4, wobei die Nanopartikel des einen oder der mehreren Zinktitanatkristalle mit einem Dispergiermittel in einer Polymermatrix dispergiert sind, die ein ölquellbares thermoplastisches Polymer in einem Träger auf Ölbasis umfasst.

6. Zusammensetzung nach Anspruch 5, wobei die Polymermatrix in der Form von Polymermatrixflocken ist, wobei jede Flocke der Polymermatrixflocken eine Flockenlänge (Lf), eine Flockenbreite (Wf) und eine Flockendicke (Tf) aufweist, wobei die Polymermatrixflocken ein dimensionsloses Flockenaspektverhältnis (Rf) aufweisen, definiert durch: Rf = (Lf•Wf)/(Tf)²; wobei in Bezug auf eine repräsentative Gruppe von zumindest zehn Polymermatrixflocken ein durchschnittliches Rf zumindest 5 beträgt; und wobei die Nanopartikel innerhalb der repräsentativen Gruppe eine kumulative Partikelgröße (D50) von höchstens 100 nm aufweisen.

7. UV-Schutzzusammensetzung nach einem von Anspruch 5 oder Anspruch 6, wobei das Dispergiermittel, das zum Dispergieren der Nanopartikel der Zinktitanatkristalle innerhalb der Polymermatrix ausgelegt ist, einen Wert für das hydrophile-lipophile Gleichgewicht (HLB) von höchstens 9 aufweist.

8. UV-Schutzzusammensetzung nach einem von Anspruch 5 bis Anspruch 7, wobei das thermoplastische Polymer in der Polymermatrix zumindest eines von einem Ethylen-Acryl-(EAA-)Polymer, einem Ethylen-Methacryl-(EMMA-)Polymer, einem Ethylvinylacetat-(EVA-)Polymer oder Kombinationen davon umfasst.

9. UV-Schutzzusammensetzung nach einem von Anspruch 1 bis Anspruch 8, formuliert als eines oder mehrere des Folgenden: (a) eine Hautpflegezusammensetzung zur Anwendung auf menschlicher oder nichtmenschlicher Tierhaut; (b) eine Haarpflegezusammensetzung zur Anwendung auf menschlichem oder nichtmenschlichem Tierhaar; oder (c) eine Beschichtungszusammensetzung zur Anwendung auf einer leblosen Oberfläche.

10. UV-Schutzzusammensetzung nach einem von Anspruch 1 oder Anspruch 2, wobei ein Molverhältnis von Ti zu Fe 19 zu 1 beträgt.

11. UV-Schutzzusammensetzung nach einem von Anspruch 1 bis Anspruch 10, wobei das Absorptionsspektrum der Zusammensetzung eine kritische Wellenlänge von zumindest 370 nm aufweist.

12. UV-Schutzzusammensetzung nach einem von Anspruch 1 bis Anspruch 11, wobei der Bereich unter der Kurve (AUC), der durch die UV-Absorption der Zinktitanatkristalle in Abhängigkeit der Wellenlänge im Bereich von 280 nm bis 400 nm (AUC₂₈₀₋₄₀₀) gebildet wird, zumindest 75 % des AUC beträgt, der durch dieselben Zinktitanatkristalle in der gleichen Konzentration im Bereich von 280 nm bis 700 nm (AUC₂₈₀₋₇₀₀) gebildet wird.

13. Verwendung der UV-Schutzzusammensetzung nach einem von Anspruch 1 bis Anspruch 12 zum Schützen des Haares eines Subjekts oder zum Schützen eines leblosen Objekts vor einer Wirkung von ultravioletter Strahlung, insbesondere zum Schützen vor ultravioletter A-Strahlung und ultravioletter B-Strahlung.

14. UV-Schutzzusammensetzung nach einem von Anspruch 1 bis Anspruch 12 zur Verwendung zum Schützen der Haut eines Subjekts vor einer Wirkung von ultravioletter Strahlung.

15. UV-Schutzzusammensetzung zur Verwendung nach Anspruch 14, wobei das Schützen vor ultravioletter Strahlung das Schützen vor ultravioletter A-Strahlung und ultravioletter B-Strahlung umfasst.

## Revendications

1. Composition de protection contre les UV comprenant un ou plusieurs cristaux de titanate de zinc possédant chacun indépendamment la formule chimique Zn₂Ti₍₁₋ₓ₎FeₓO₄, x étant compris entre 0,005 et 0,1, lesdits cristaux de titanate de zinc étant sous forme de nanoparticules comprenant un ou plusieurs desdits cristaux, au moins 50 % du nombre total desdites nanoparticules possédant au moins une dimension allant jusqu'à 200 nm.

2. Composition de protection contre les UV selon la revendication 1, x étant compris entre 0,025 et 0,05.

3. Composition de protection contre les UV selon l'une quelconque des revendications 1 ou 2, au moins 90 % du nombre total desdites nanoparticules possédant au moins une dimension allant jusqu'à environ 200 nm.

4. Composition de protection contre les UV selon l'une quelconque des revendications 1 à 3, ledit ou lesdits cristaux de titanate de zinc étant à une concentration comprise dans la plage allant d'environ 0,001 % à environ 40 % (p/p) de la composition.

5. Composition de protection contre les UV selon l'une quelconque des revendications 1 à 4, lesdites nanoparticules dudit ou desdits cristaux de titanate de zinc étant dispersées avec un dispersant dans une matrice polymère comprenant un polymère thermoplastique gonflable dans l'huile dans un support à base d'huile.

6. Composition selon la revendication 5, ladite matrice polymère étant sous la forme de flocons de matrice polymère, chaque flocon desdits flocons de matrice polymère possédant une longueur de flocon (Lf), une largeur de flocon (Wf) et une épaisseur de flocon (Tf), lesdits flocons de matrice polymère possédant un rapport d'aspect des flocons sans dimension (Rf) défini par : Rf = (Lf•Wf)/(Tf)² ; par rapport à un groupe représentatif d'au moins dix flocons de matrice polymère, un Rf moyen étant d'au moins 5 ; et lesdites nanoparticules dans ledit groupe représentatif possédant une taille de particule cumulative (D50) inférieure ou égale à 100 nm.

7. Composition de protection contre les UV selon l'une quelconque des revendications 5 ou 6, ledit dispersant adapté pour disperser les nanoparticules des cristaux de titanate de zinc dans ladite matrice polymère possédant une valeur d'équilibre hydrophile-lipophile (HLB) inférieure ou égale à 9.

8. Composition de protection contre les UV selon l'une quelconque des revendications 5 à 7, ledit polymère thermoplastique dans la matrice polymère comprenant au moins l'un d'un polymère d'éthylène-acrylique (EAA), un polymère d'éthylène-méthacrylique (EMMA), un polymère d'acétate d'éthyle vinyle (EVA) ou des combinaisons de ceux-ci.

9. Composition de protection contre les UV selon l'une quelconque des revendications 1 à 8, formulée telle qu'un ou plusieurs des suivants : (a) une composition de soin de la peau pour application sur la peau d'animaux humains ou non humains ; (b) une composition de soin capillaire à appliquer sur des poils d'animaux humains ou non humains ; ou (c) une composition de revêtement en vue d'une application sur une surface inanimée.

10. Composition de protection contre les UV selon l'une quelconque des revendications 1 ou 2, un rapport molaire de Ti sur Fe étant de 19 à 1.

11. Composition de protection contre les UV selon l'une quelconque des revendications 1 à 10, ledit spectre d'absorption de la composition possédant une longueur d'onde critique supérieure ou égale à 370 nm.

12. Composition de protection contre les UV selon l'une quelconque des revendications 1 à 11, ladite aire sous la courbe (AUC) formée par l'absorption d'UV des cristaux de titanate de zinc en fonction de la longueur d'onde comprise dans la plage allant de 280 nm à 400 nm (AUC₂₈₀₋₄₀₀) étant supérieure ou égale à 75 % de l'AUC formée par les mêmes cristaux de titanate de zinc à la même concentration comprise dans la plage allant de 280 nm à 700 nm (AUC₂₈₀₋₇₀₀).

13. Utilisation de la composition anti-UV selon l'une quelconque des revendications 1 à 12, en vue de la protection des poils d'un sujet ou en vue de la protection un objet inanimé contre un effet des rayons ultraviolets, en particulier la protection contre le rayonnement ultraviolets A et le rayonnement ultraviolets B.

14. Composition de protection contre les UV selon l'une quelconque des revendications 1 à 12, destinée à être utilisée pour protéger la peau d'un sujet contre un effet du rayonnement ultraviolet.

15. Composition de protection contre les UV destinée à être utilisée selon la revendication 14, ladite protection contre le rayonnement ultraviolet comprenant la protection contre le rayonnement ultraviolet A et le rayonnement ultraviolet B.
